# EUROPEAN PATENT SPECIFICATION

(11) **EP 4 549 952 B1**
(45) Date of publication and mention of the grant of the patent: **02.12.2026**
(21) Application number: 24209163.5
(22) Date of filing: 28.10.2024
(51) Int. Cl.: G01N 33/487, G01N 35/04, G01N 33/493

(54) **ANALYZER**
ANALYSATOR
ANALYSEUR

(30) Priority: 30.10.2023 JP 2023186024; 18.10.2024 JP 2024184230
(43) Date of publication of application: 07.05.2025
(60) Divisional application: 26208143.3 / 4 838 217
(73) Proprietor: ARKRAY, Inc., Kyoto-shi, Kyoto 601-8045 (JP)
(72) Inventor: FURUSATO, Noriaki, Kyoto-shi, 602-0008 (JP)
(74) Representative: MFG Patentanwälte Meyer-Wildhagen Meggle-Freund Gerhard PartG mbB

(56) References cited:
- WO-A1-2011/122562
- JP-A- 2001 153 874
- JP-A- 2005 345 253
- US-A1- 2013 152 709

## Description

### TECHNICAL FIELD

The present disclosure relates to an analyzer that analyzes a sample using an analysis instrument such as a test paper.

### BACKGROUND ART

The related art discloses an analyzer for analyzing the presence or absence, concentration, and the like of a specific component in a sample by optically measuring a color development reaction in a reagent unit on a test paper (see International Patent Publication No. WO2011/122562 (hereinafter, referred to as Patent Literature 1) and Japanese Patent Application Laid-Open Publication No. 2005-345253). In analysis, a certain amount of time is required from deposition of the sample to measurement because of the wait for the color development caused by the chemical reaction between the reagent unit impregnated with the reagent and the sample.

As described above, a certain amount of time is needed before the color development can be measured optically.

Here, if the time required for observation is attempted to be secured by lengthening a horizontal conveying path from a position where the deposition is performed to a position where the reading is performed, the width of the analyzer will increase. In some cases, this will interfere with work in a space where the analyzer is installed.

US 2013152709 discloses a test piece transfer apparatus including a transfer mechanism for transferring a test piece in a transfer direction. It is disclosed that a movement path of the transfer mechanism from a receiving point to a position control finishing point is inclined with respect to an x-direction.

The present disclosure describes a technique to suppress increase in horizontal size of a housing of an analyzer.

### SUMMARY OF INVENTION

An aspect of the present disclosure relates to an analyzer that deposits a sample on a test paper to analyze a specific component in the sample. The analyzer includes: a deposition device configured to deposit the sample on the test paper; a reading device configured to read optical information of the test paper on which the sample is deposited; a conveying mechanism configured to convey the test paper from a deposition position at which deposition is performed by the deposition device to a reading position at which reading by the reading device is performed; and a housing configured to accommodate the deposition device, the reading device, and the conveying mechanism. The reading position is in a higher position than the deposition position, and the conveying mechanism is configured to convey the test paper obliquely upward.

With the configuration described above, the conveying mechanism extends obliquely upward, and not only a horizontal space but also a vertical space is effectively utilized to provide time for observing color development. Therefore, it becomes possible to suppress horizontal size of the housing of the analyzer increasing.

The analyzer according may further include: a disposal box into which the test paper is put after the reading device reads the test paper; and a take-out port of the disposal box that is provided on a front surface of the housing. The disposal box is removable from the take-out port. With this configuration, the disposal box can be taken out from the take-out port on the front surface of the housing, and another analyzer or equipment can be installed in the vicinity of side of the analyzer. In the configuration in which the disposal box is taken out from the front surface, it is easy to avoid interference with various devices in the housing, and an increase in size of the analyzer can be prevented.

A plurality of door members may be provided on the front surface of the housing, the plurality of door members corresponding to the take-out port and being openable and closable around an axis extending in different directions. According to this configuration, a door member can be closed when the analyzer is operated, and each door member can be opened when the disposal box is taken out. Since the door members can be opened in different directions, the take-out port and a path (for example, a path directed forward and obliquely upward) can be easily secured.

The plurality of door members may include a first door member and a second door member, the first door member may be configured to open and close the housing by rotating around an axis extending in an up-down direction, and the second door member may be configured to take a standing posture in the up-down direction by rotating around an axis extending in a left-right direction. With this configuration, it becomes easy to secure the take-out port and a path (for example, a path extending forward and obliquely upward).

The deposition device may have a nozzle configured to collect the sample from a container accommodating the sample to deposit the sample on the test paper, and the nozzle may be arranged in a position that does not overlap the plurality of door members in a front view. It becomes possible to prevent taking out and accommodating of the disposal box from interfering with an area within which the nozzle operates.

The analyzer may further include a placement table on which the container is placed. The disposal box may be removable from the take-out port via a path passing above the container on the placement table. A container may be placed on the placement table in front of the housing. In this case, since the disposal box can be taken out along a path passing above the container, and thus interference with the container can also be prevented. Accordingly, it becomes possible to take out the disposal box from the housing without any trouble while preventing an increase in size of the analyzer.

The analyzer may further include a sensor configured to detect an amount of test papers in the disposal box. The deposition device may be configured to perform a predetermined notification when the sensor detects that the amount of test papers in the disposal box is more than a predetermined amount. A user can know a timing at which the disposal box should be taken out.

### BRIEF DESCRIPTION OF DRAWINGS

Exemplary embodiments of the present disclosure will be described in detail based on the following figures, wherein:
Fig. 1 is a perspective view showing an analyzer according to an embodiment of the present disclosure;
Fig. 2 is a front view showing a schematic configuration of an inside of a device main body in Fig. 1;
Fig. 3 is a perspective cross-sectional view showing first and second conveying mechanisms, a deposition device, a reading device, and a disposal box in the device main body;
Fig. 4 is a block diagram showing a configuration of the reading device and a test unit;
Fig. 5 is a perspective view showing a drive unit and a movable plate of the second conveying mechanism;
Fig. 6A is a front view of an outer fixing plate;
Fig. 6B is a front view of the movable plate;
Fig. 7 is a perspective cross-sectional view showing the reading position and the disposal box located at an uppermost portion of the second conveying mechanism;
Fig. 8A is a front view showing a procedure of discharging test papers in a discharge unit;
Fig. 8B is a front view showing a procedure of discharging test papers in the discharge unit;
Fig. 8C is a front view showing a procedure of discharging test papers in the discharge unit;
Fig. 8D is a front view showing a procedure of discharging test papers in the discharge unit;
Fig. 9 is a side sectional view showing a take-out port of the disposal box and a path.
Fig. 10 is a diagram showing an analyzer according to a first modification; and
Fig. 11 is a diagram showing an analyzer according to a second modification.

### DESCRIPTION OF EMBODIMENTS

Hereinafter, embodiments of the present invention will be described with reference to the drawings. The same elements are denoted by the same reference numerals, and redundant descriptions thereof will be omitted. The dimensional ratios in the drawings do not necessarily coincide with those in the description. In the following embodiments and modifications, a urine test device will be described as an example of an analyzer.

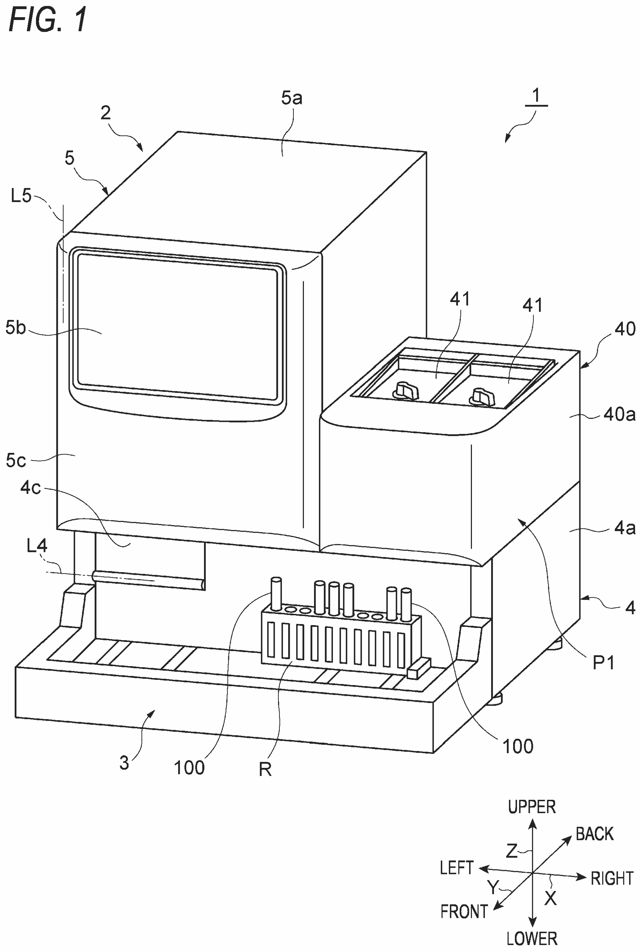

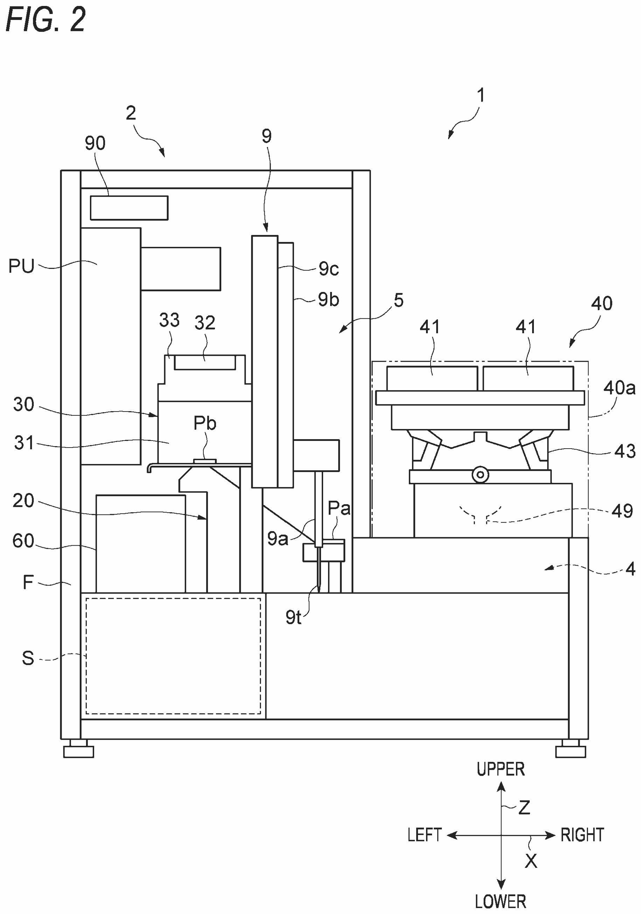

In some of the drawings, an X-direction, a Y-direction, and a Z-direction orthogonal to each other are shown, and each of these directions may be used to describe a urine test device 1. An X-direction, a Y-direction, and a Z-direction respectively correspond to a left-right direction, a front-rear direction, and an up-down direction in an installation state of the urine test device 1 shown in Figs. 1 and 2. In the present specification, terms indicating directions such as "front", "rear", "up", "down", "left", and "right" are terms based on an installation state of the urine test device 1. The urine test device 1 is installed on a flat and horizontal surface. "Left" and "right" are directions when the urine test device 1 is viewed from an operator who stands in front of a placement table 3 and a display 5b and faces the placement table 3 and the display 5b.

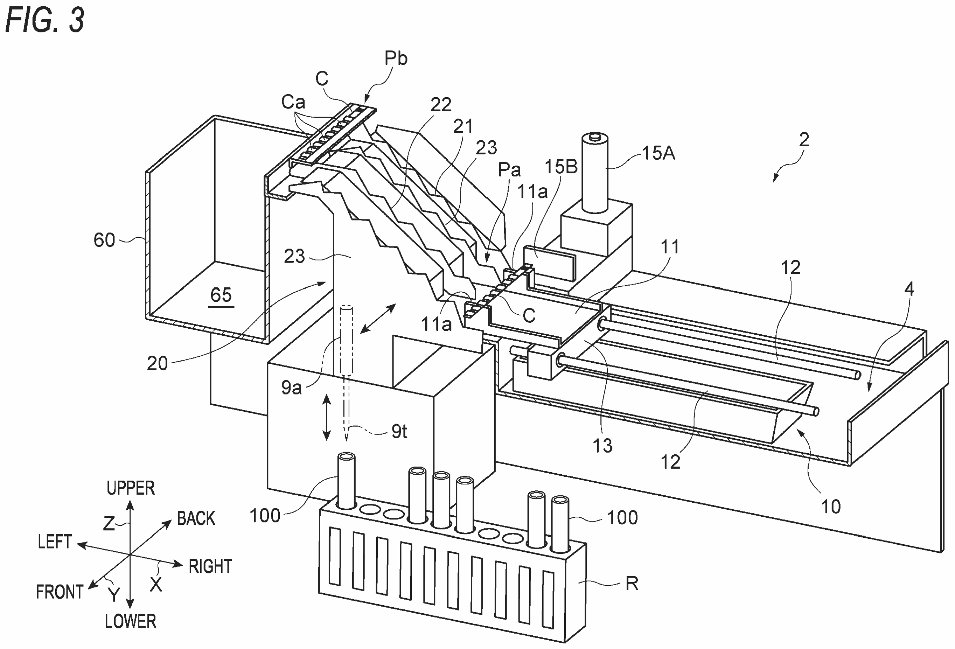

First, an overall configuration of the urine test device 1 will be described with reference to Figs. 1 and 2. The urine test device 1 is a device for testing urine as a sample accommodated in a spit (a container) 100. As shown in Fig. 1, the urine test device 1 sequentially tests samples in a plurality of spits 100 standing on a rack R. Test items (analysis items) are not particularly limited. Examples of the test items include physical properties of the sample and the presence or absence or concentration of a specific component in the sample. In the urine test device 1, an elongated test paper C is used for the test of the sample (see Fig. 3). One test paper C is used for one sample. Each test paper C is provided with, for example, a plurality of reagent units Ca. Each reagent unit Ca may correspond to different test items. Each reagent unit Ca includes a reagent corresponding to a test item. A known configuration may be adopted as the test paper C.

As shown in Fig. 1, the urine test device 1 includes a device main body 2 in which various devices and mechanisms related to the conveyance of the test paper C and the test of the sample are accommodated, and the placement table 3 including a conveying mechanism and the like related to the supply and conveyance of the rack R. The device main body 2 includes a device lower portion 4 and a device upper portion 5 disposed on a portion (for example, a left side) of the device lower portion 4 in the left-right direction. The placement table 3 is coupled to a front surface of the device lower portion 4. The urine test device 1 further includes a supply mechanism 40, which is a mechanism for accommodating the test paper C and supplying the test paper C to the device main body 2. The supply mechanism 40 is disposed on another portion (for example, a right side) of the device lower portion 4 in the left-right direction.

As shown in Fig. 2, the device main body 2 is adjacent to the rear of the placement table 3. The device main body 2 includes a frame F formed of structural members that extend in the up-down direction, the left-right direction, and the front-rear direction. As shown in Fig. 1, the device lower portion 4 includes a lower housing 4a, and the device upper portion 5 includes an upper housing 5a. The lower housing 4a and the upper housing 5a form a rectangular parallelepiped shape based on a shape of the frame F. A front panel (a door member) 5c that is openable and closable by rotating around an axis L5 extending in the up-down direction (the Z-direction), for example, is provided on a front surface of the upper housing 5a. A display 5b is provided on a front panel 5c. The display 5b is directed forward and displays information necessary for a test to an operator of the urine test device 1. The display 5b may have a touch panel function. In this case, the display 5b can also serve as an operation unit operated by the operator.

The placement table 3 has substantially the same width as the device main body 2 in the left-right direction. The placement table 3 has a predetermined size in plan view. A plurality of racks R can be placed on the placement table 3. Although only one rack R is shown in Fig. 1, a plurality of racks R can be placed on a right side and a left side of the placement table 3. The placement table 3 is provided with a conveying mechanism (not shown) that conveys the rack R in a necessary direction. As a specific configuration of the conveying mechanism, a known configuration can be adopted. As an example, devices (structures) described in Japanese Patent Application Laid-Open Publication No. H10-120167 and Japanese Patent Application Laid-Open Publication No. H10-139152 may be applied.

As described above, each spit 100 accommodates a sample. The plurality of spits 100 are placed on the placement table 3 via the rack R. The plurality of spits 100 can be placed at any position in the left-right direction by being moved in the left-right direction by the conveying mechanism.

Returning to Fig. 2, in the device main body 2, for example, a controller 90 is provided in the upper housing 5a. The controller 90 is, for example, a computer including a read only memory (ROM) in which a program or the like is stored, a storage medium such as a random access memory (RAM) in which data is temporarily stored, and a processor such as a central processing unit (CPU). The controller 90 controls operations of a mechanism and a device in the urine test device 1 in accordance with a program stored in advance in response to an operation by the operator, and performs various operations related to the test. The controller 90 can communicate information with various motors and centers included in the supply mechanism 40, a first conveying mechanism 10, and a second conveying mechanism 20, a camera 32, an illuminator 33, and the like of an imaging device 30.

As shown in Figs. 1 and 2, the supply mechanism 40 is a feeder for supplying the test papers C one by one in a predetermined direction (posture). The supply mechanism 40 includes a rectangular parallelepiped housing 40a, a casing 43 disposed inside the housing 40a, and a pair of input lids 41 that are openable and closable with respect to the casing 43. The supply mechanism 40 accommodates therein a plurality of test papers C input by the operator through the input lids 41. The plurality of test papers C are put in a state in which directions in a length direction are aligned (in directions shown in Fig. 3) such that portions in which the plurality of reagent units Ca (see Fig. 3) are arranged at regular intervals are disposed in front. The supply mechanism 40 sequentially discharges the test papers C one by one from a discharge unit 49. In the supply mechanism 40, a sensor (not shown) is provided to detect that one test paper C has been successfully taken out. When the controller 90 detects that the test paper C is to be supplied (the test paper C is to be discharged from the discharge unit 49), the controller 90 controls the first conveying mechanism 10 (see Fig. 3) to be described later to move an arm 11 to a position immediately below the discharge unit 49 at a predetermined timing. The test paper C dropped from the discharge unit 49 is placed on a support portion 11a of the arm 11. Accordingly, the supply mechanism 40 sequentially supplies the test papers C one by one to the first conveying mechanism 10.

In the present embodiment, the supply mechanism 40 includes the pair of input lids 41 and can supply two types (many types) of test papers C. This is merely an example, and the supply mechanism 40 may be of a type capable of supplying only one type of test paper C.

Next, various configurations provided in the device main body 2 will be described with reference to Figs. 2 and 3. The device main body 2 includes the first conveying mechanism 10 that conveys the test paper C received from the supply mechanism 40 to a deposition position Pa or the vicinity thereof, a deposition device 9 that deposits a sample on each reagent unit Ca of the test paper C disposed at the deposition position Pa, the second conveying mechanism 20 that conveys the test paper C from the deposition position Pa to an imaging position (a reading position) Pb, and the imaging device (a reading device) 30 that images the test paper C disposed at the imaging position Pb. That is, the device main body 2 deposits a sample on the test paper C and analyzes a specific component in the sample.

The device main body 2 further includes a disposal box 60 that stores the test paper C released (or discharged) from the imaging device 30 after the imaging device 30 has finished imaging. The rectangular parallelepiped disposal box 60 is disposed on a left side of the imaging device 30. The disposal box 60 is open upward, and can store a large number of (a certain number of) test papers C in a storage space 65 inside. In the present embodiment, for example, an internal space S (see Fig. 1) having a size (a volume) equal to or larger than that of the disposal box 60 is formed below the disposal box 60.

As shown in Fig. 3, the first conveying mechanism 10 includes, for example, the arm 11 that supports the test paper C by the support portion 11a, a moving block 13 to which the arm 11 is fixed, and a pair of shafts 12 that extend in the X-direction (a horizontal direction) and guide the moving block 13 in the X-direction. Further, the first conveying mechanism 10 includes a drive motor controlled by the controller 90, a pair of pulleys, a timing belt stretched over the pair of pulleys, and the like (all not shown). The moving block 13 is connected to the timing belt. The first conveying mechanism 10 moves the arm 11 to a position directly below the discharge unit 49 at the predetermined timing. The first conveying mechanism 10 receives one test paper C from the supply mechanism 40, and then conveys the test paper C to a predetermined position above the deposition position Pa where the sample is deposited. That is, in the present embodiment, the first conveying mechanism 10 conveys the test paper C received from the supply mechanism 40 to the vicinity of the deposition position Pa.

A rear end of the test paper C conveyed above the deposition position Pa is aligned in a front direction with an extruded plate 15A movable in the front-rear direction. The movement of the extruded plate 15A is adjusted by converting a rotational driving force of an extrusion motor 15B into a straight line by a crank mechanism (not shown). Other known configurations may be appropriately adopted for a mechanism for driving the extruded plate 15A.

The first conveying mechanism 10 is not limited to the above configuration. The first conveying mechanism 10 may convey, to the deposition position Pa, the test paper C received from the supply mechanism 40. The first conveying mechanism 10 may be any mechanism that can receive the test paper C from the supply mechanism 40 and convey the test paper C in a lateral direction. Other known configurations may be adopted as the first conveying mechanism 10. For example, the first conveying mechanism 10 may include a belt conveyor or the like. The first conveying mechanism 10 may include a slide rail, a linear guide, or the like.

As shown in Figs. 2 and 3, the deposition device 9 collects (samples) a predetermined amount of the sample in the spit 100, and deposits the sample on each reagent unit Ca of the test paper C disposed at the deposition position Pa. The deposition device 9 includes a support drive mechanism 9c and a rail 9b disposed in the upper housing 5a, and a nozzle 9a movable in the up-down direction (the Z-direction) and the front-rear direction (the Y-direction) along the rail 9b. The support drive mechanism 9c includes a drive motor (not shown). When the controller 90 detects that the spit 100 arrives at a predetermined position in front of the deposition device 9 (in front of the deposition position Pa), the deposition device 9 is controlled by the controller 90, and the sample is collected by the nozzle 9a. When the nozzle 9a moves forward, the nozzle 9a reaches a position protruding from the device main body 2 (that is, a region of the placement table 3). That is, the nozzle 9a is movable between the device main body 2 and the placement table 3. When the sample is collected, a pump unit PU disposed in the upper housing 5a is also controlled by the controller 90 and operates in conjunction with the deposition device 9.

The deposition position Pa is set at a position slightly higher than an upper end of the spit 100 on the rack R. Accordingly, a stroke in the up-down direction of the nozzle 9a is a minimum distance. That is, a tip 9t of the nozzle 9a needs to enter a predetermined depth in the spit 100, but after the sample is collected, the tip 9t separates from the spit 100 and moves backward at a position slightly higher than an upper end thereof. Thereafter, for example, deposition is performed on each reagent unit Ca in order from the rear reagent unit Ca. The deposition device 9 repeats the same operation every time a new sample (the spit 100) arrives at the predetermined position. Although not shown, the deposition device 9 includes a cleaning unit or the like that cleans the nozzle 9a between the deposition position Pa and the spit 100.

As shown in Figs. 2 and 3, the second conveying mechanism 20 is, for example, a mechanism that conveys the plurality of test papers C stepwise at a predetermined pitch. In the present embodiment, the imaging position Pb is located on the left of the deposition position Pa and is high. The second conveying mechanism 20 conveys the test paper C obliquely upward. The second conveying mechanism 20 includes, for example, a pair of outer fixing plates 21, a single central fixing plate 22 disposed at an intermediate position between the pair of outer fixing plates 21 in the front-rear direction, and a pair of movable plates 23 disposed between the pair of outer fixing plates 21 and the central fixing plate 22 (since Fig. 3 is a cross-sectional view, the front outer fixing plate 21 is not shown). The outer fixing plates 21, the central fixing plate 22, and the movable plates 23 are disposed parallel to an XZ plane and form a substantially congruent step shape. In the outer fixing plates 21, the central fixing plate 22, and the movable plates 23, all the horizontal step portions are set to have the same pitch (a height difference and a distance in the left-right direction).

The pair of movable plates 23 are moved by a drive motor (not shown) to draw a predetermined circulating orbit. The pair of movable plates 23 move along a substantially rectangular circulating orbit (a counterclockwise orbit when viewed from the front) so as to move between two adjacent horizontal step portions in the outer fixing plates 21 and the central fixing plate 22. As a result, the second conveying mechanism 20 conveys the test paper C placed on a horizontal step portion to a horizontal step portion immediately above, and repeats the operation.

The test paper C conveyed by the first conveying mechanism 10 above the deposition position Pa is conveyed from the arm 11 of the first conveying mechanism 10 to the deposition position Pa by an operation of the movable plate 23. That is, the test paper C conveyed to the delivery position is lifted upward from the arm 11 by the movable plate 23. At this time, the arm 11 moves to a position immediately below the discharge unit 49 (for conveying the next test paper C). The test paper C lifted upward is placed on the lowest horizontal step portion of the outer fixing plates 21 and the central fixing plate 22, which are the deposition positions Pa, while drawing a substantially rectangular circulating orbit (a clockwise orbit when viewed from the front). The uppermost horizontal step portion of the outer fixing plates 21 and the central fixing plate 22 is disposed inside the imaging device 30 and is the imaging position Pb. Conveyance of the second conveying mechanism 20 is performed in consideration of a time required for color development of the reagent unit Ca. Details of the second conveying mechanism 20 will be described later.

The second conveying mechanism 20 is not limited to the above configuration. The second conveying mechanism 20 in the present embodiment only needs to convey the test paper C disposed at the deposition position Pa obliquely upward. Other known configurations may be adopted as the second conveying mechanism 20. For example, the second conveying mechanism 20 may include a belt conveyor or the like.

As shown in Fig. 1, the imaging device 30 includes an imaging chamber 31 having a rectangular parallelepiped shape (or a cubic shape) and disposed to surround the imaging position Pb, the camera 32 disposed above the imaging chamber 31, and the illuminator 33 disposed on the imaging chamber 31 and around the camera 32. The imaging device 30 reads optical information on the test paper C on which the sample is deposited. The imaging position Pb is located substantially at a center of the imaging chamber 31 and at a bottom of the imaging chamber 31. The test paper C is supported by the arm 11 of the first conveying mechanism 10, and maintains a posture in which a length direction of the test paper C is oriented in the front-rear direction (a direction perpendicular to the plane of Fig. 1) and the reagent unit Ca faces upward until the test paper C reaches the imaging position Pb by being conveyed by the second conveying mechanism 20. The camera 32 images the entire test paper C and transmits image information to the controller 90.

The controller 90 performs a test relating to each test item, that is, measurement and determination based on the image of the test paper C obtained by the imaging device 30. The controller 90 causes the display 5b to display a measurement result and a determination result together with unique information such as a sample ID. The controller 90 stores the measurement result and the determination result in a storage unit together with unique information such as the sample ID. The controller 90 has a function of a test unit in the urine test device 1.

The test paper C that has been captured by the imaging device 30 is released (discharged) from the imaging device 30 by the second conveying mechanism 20, and is put into the disposal box 60. The first conveying mechanism 10, the second conveying mechanism 20, the deposition device 9, and the disposal box 60 are accommodated in the lower housing 4a or accommodated in the lower housing 4a and the upper housing 5a.

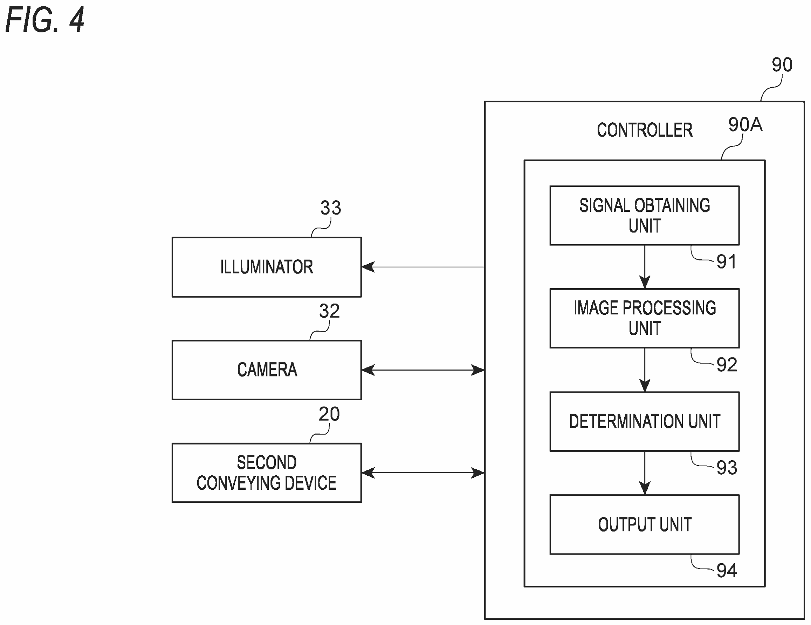

Next, a function of a test unit 90A provided in the controller 90 will be described with reference to Fig. 4. The controller 90 can control the illuminator 33, the camera 32, and the second conveying mechanism 20. Further, the controller 90 can input a captured image (a read image) obtained by the camera 32. When the test paper C is conveyed to the imaging position Pb, the controller 90 controls the camera 32 and the illuminator 33 to image the test paper C. At this time, the captured image includes color development results of the plurality of reagent units Ca. The controller 90 functions as the test unit 90A. The test unit 90A includes a signal obtaining unit 91 that obtains a signal corresponding to a captured image, an image processing unit 92 that performs predetermined image processing, a determination unit 93 that performs determination related to the test items based on a result of the image processing, and an output unit 94 that outputs a determination result (or a measurement result) and displays the determination result on the display 5b (and stores in the storage unit). The determination unit 93 may store a threshold, a calculation formula, or the like corresponding to the test items.

Next, a detailed configuration of the second conveying mechanism 20, the arrangement of the imaging device 30 (the imaging position Pb) and the disposal box 60 installed to be continuous to a side of the second conveying mechanism 20, and the like will be described. Depending on the test items (types), a test device different from the urine test device 1 may be required. For example, another analyzer (not shown) may be installed on a left side of the urine test device 1. The other analyzer is installed adjacent to the urine test device 1. In this case, the placement table 3 of the urine test device 1 may be connected to a placement table of another test device, and the rack R and the plurality of spits 100 may be transferred from the placement table 3 to another placement table. The rack R and the plurality of spits 100 may pass through a front position of the second conveying mechanism 20 and the internal space S, or may stop at the front position.

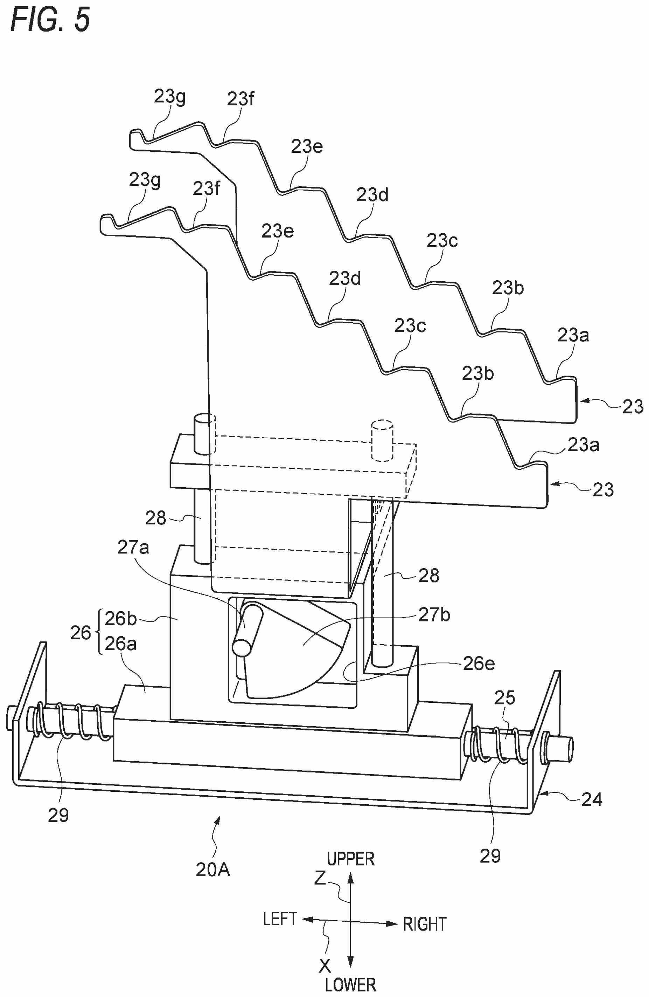

The urine test device 1 according to the present embodiment has a configuration in which the disposal box 60 can be taken out from the front of the upper housing 5a and the lower housing 4a even when the rack R and the plurality of spits 100 are present at the front position. Hereinafter, the configuration will be described with reference to Figs. 3 and 5.

As shown in Fig. 3, the imaging position Pb where the test paper C is disposed in the imaging device 30 is set to a position higher than the deposition position Pa where the test paper C is disposed in the deposition device 9. As described above, the second conveying mechanism 20 conveys the plurality of test papers C stepwise at a predetermined pitch. The second conveying mechanism 20 conveys the test paper C obliquely upward.

As shown in Fig. 5, the second conveying mechanism 20 includes a drive unit 20A that moves the pair of movable plates 23. The drive unit 20A includes a fixed base 24 fixed to the lower housing 4a, left and right guide shafts 25 provided on the fixed base 24 and extending in the left-right direction, and a slide block 26a movable in the left-right direction along the left and right guide shafts 25. A pair of left and right biasing springs 29 are provided between the fixed base 24 and the slide block 26a. For example, the biasing springs 29 each formed of a compression coil spring bias the slide block 26a to return to a center position in the left-right direction in a state where the left and right guide shafts 25 are inserted. The drive unit 20A further includes a pair of upper and lower guide shafts 28 erected on the slide block 26a, and an elevating block 26b movable in the up-down direction along the upper and lower guide shafts 28. The slide block 26a and the elevating block 26b constitute a movable portion 26. The pair of movable plates 23 are fixed on the elevating block 26b.

A rectangular hole 26e penetrating in the front-rear direction is formed in the elevating block 26b. The drive unit 20A further includes a camshaft 27a that is disposed in the rectangular hole 26e and extends in the front-rear direction, and a fan-shaped cam 27b that rotates together with the fan-shaped cam 27b in the rectangular hole 26e. The camshaft 27a is fixed to the lower housing 4a via a bracket or the like (not shown). The drive unit 20A rotates the fan-shaped cam 27b by rotating the camshaft 27a with a drive motor (not shown). An arc-shaped end surface of the fan-shaped cam 27b is normally in contact with a wall surface of the rectangular hole 26e. As the fan-shaped cam 27b rotates, the slide block 26a slides in the left-right direction, and the elevating block 26b moves (raises and lowers) in the up-down direction. By the sliding movement of the slide block 26a and the movement (raise and lower) of the elevating block 26b, the pair of movable plates 23 move along a substantially rectangular circulating orbit. The movement of the pair of movable plates 23 is a translational motion parallel to the XZ plane. As described above, in the drive unit 20A, the pair of movable plates 23 are moved using a cam mechanism including the fan-shaped cam 27b as a driving link and the elevating block 26b as a driven link. The drive motor can rotate the camshaft 27a in both directions. The fan-shaped cam 27b is rotatable in a counterclockwise forward direction when viewed from the front, and is rotatable in a clockwise reverse direction when viewed from the front.

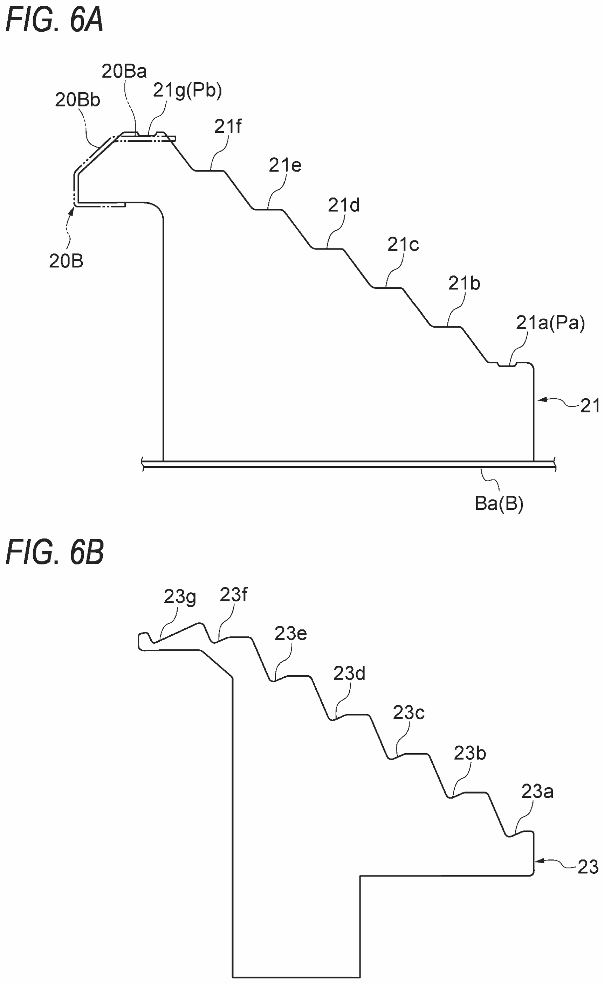

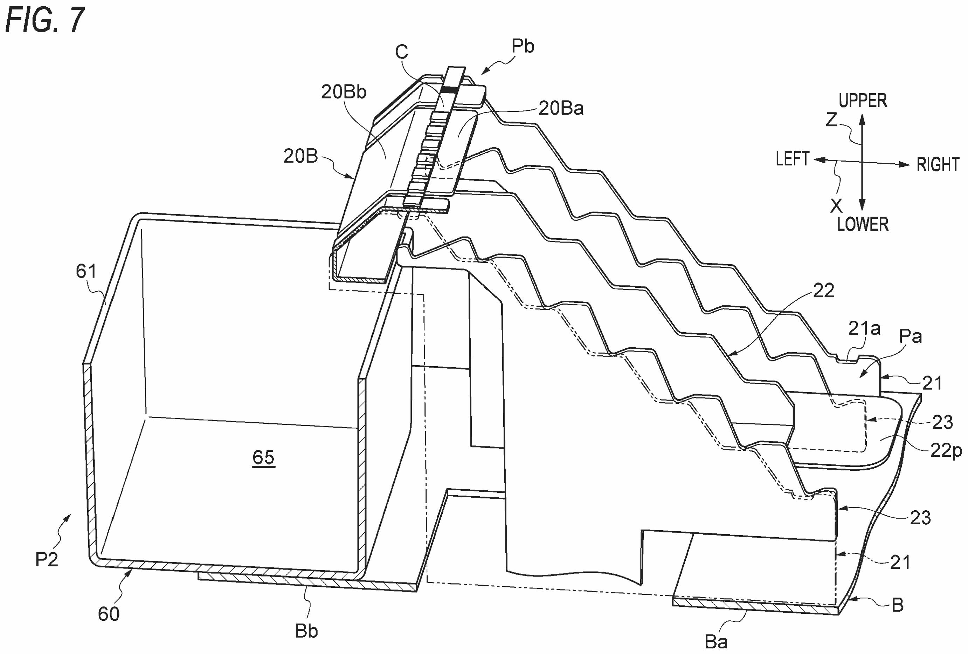

As shown in Fig. 6A, the outer fixing plates 21 are fixed to a plate-shaped common base B extending horizontally. The outer fixing plates 21 are fixed to a first support portion Ba (see Fig. 7) which is a right side portion of the common base B. Each of the outer fixing plates 21 includes a plurality of horizontal step portions. More specifically, the outer fixing plate 21 includes, for example, a first horizontal step portion 21a, a second horizontal step portion 21b, a third horizontal step portion 21c, a fourth horizontal step portion 21d, a fifth horizontal step portion 21e, a sixth horizontal step portion 21f, and a seventh horizontal step portion 21g. The horizontal step portions have the same shape except for the first horizontal step portion 21a and the seventh horizontal step portion 21g. Only the first horizontal step portion 21a and the seventh horizontal step portion 21g are formed in a recessed shape. In the left-right direction, a size of each horizontal step portion is larger than a width of the test paper C. The test paper C is placed on each horizontal step portion. Pitches (a height difference and a distance in the left-right direction) of the plurality of horizontal step portions are set to be equal.

As described above, the lowermost first horizontal step portion 21a defines the deposition position Pa. A horizontal support plate 22p (see Fig. 7) is fixed to a lowermost portion of the central fixing plate 22, and the support plate 22p also supports the test paper C at the deposition position Pa. The uppermost seventh horizontal step portion 21g defines the imaging position Pb.

As shown in Figs. 5 and 6B, each movable plate 23 includes a plurality of moving step portions. More specifically, the movable plate 23 includes, for example, a first moving step portion 23a, a second moving step portion 23b, a third moving step portion 23c, a fourth moving step portion 23d, a fifth moving step portion 23e, a sixth moving step portion 23f, and a seventh moving step portion 23g. These moving step portions also have the same shape. Each moving step portion is slightly inclined with respect to a horizontal plane (the XY plane), and descends toward the left. In the left-right direction, a size of each moving step portion is equal to or slightly smaller than that of each horizontal step portion. The test paper C is placed on each moving step portion. Due to an inclined shape of each moving step portion, a position and posture of the test paper C are stabilized while the test paper C is conveyed by the pair of movable plates 23. Pitches (a height difference and a distance in the left-right direction) of the plurality of moving step portions are set to be equal.

As shown in Figs. 6A and 6B, a shape and size of the movable plate 23 conform to a shape and size of the outer fixing plate 21 (and the central fixing plate 22 (see Fig. 7)). When the movable plate 23 is raised or lowered at a right end position of a movable range, each of all the movable step portions passes through each position of all the horizontal step portions when viewed from the front. Further, when the movable plate 23 is raised or lowered at a left end position (a position close to the disposal box 60) of a movable range, the first moving step portion 23a to the sixth moving step portion 23f pass through a position of the second horizontal step portion 21b to the seventh horizontal step portion 21g when viewed from the front, respectively. That is, the movable plate 23 moves (shifts) in the front-rear direction by a length corresponding to one pitch.

As shown in Figs. 6A and 7, a discharge unit 20B that discharges the test paper C into the disposal box 60 is provided at a position at a left end and an upper end of the second conveying mechanism 20. That is, the second conveying mechanism 20 includes the discharge unit 20B for the test paper C. The discharge unit 20B discharges the test paper C that has been captured (read) by the imaging device 30. That is, the discharge unit 20B may be referred to as a "final discharge unit" that finally discharges the captured test paper C to the disposal box 60. As shown in Fig. 7, the discharge unit 20B is disposed at a position higher than an upper-end edge 61 of the disposal box 60 installed at a take-out position P2. In Fig. 6A, the discharge unit 20B is indicated by an imaginary line. The discharge unit 20B includes a horizontal plate-shaped placement surface 20Ba and a plate-shaped discharge surface 20Bb inclined from the placement surface 20Ba to a left lower side. A height of an upper surface of the placement surface 20Ba coincides with a height of a bottom surface of the seventh horizontal step portion 21g. A lower end of the discharge surface 20Bb faces the storage space 65 of the disposal box 60.

The discharge unit 20B is provided, for example, at a position below the imaging device 30. The placement surface 20Ba of the discharge unit 20B also serves as an imaging surface of the imaging device 30. In other words, the placement surface 20Ba provides the imaging position Pb of the test paper C together with the seventh horizontal step portion 21g of the outer fixing plate 21.

As shown in Fig. 7, the disposal box 60 is placed on the second support portion Bb which is a left side portion of the horizontal plate-shaped common base B. The second support portion Bb extends, for example, at a height position of an upper end of the internal space S (see Fig. 2) described above.

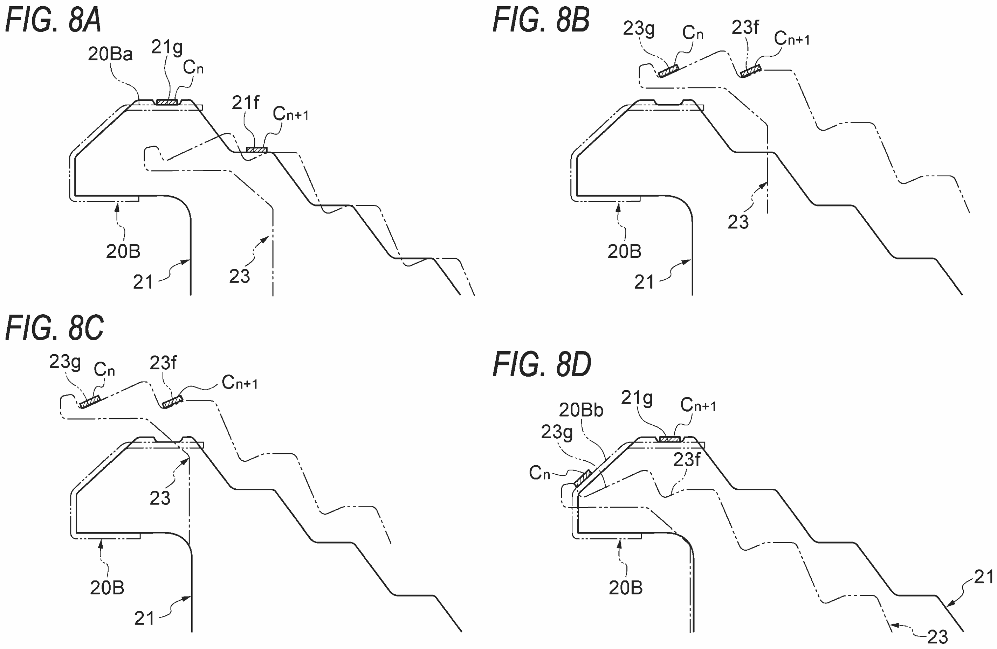

The conveyance of the test paper C by the second conveying mechanism 20 and the discharge of the test paper C by the discharge unit 20B will be described with reference to Figs. 8A to 8D. As shown in Fig. 8A, for example, a test paper Cₙ is placed on the seventh horizontal step portion 21g, and a test paper C_{n + 1} is placed on the sixth horizontal step portion 21f. The test paper Cₙ is captured (optically read) by the imaging device 30. Thereafter, as shown in Fig. 8B, the pair of movable plates 23 are raised at the right end position of the movable range. The test paper Cₙ is supported by the seventh moving step portion 23g, and the test paper C_{n + 1} is supported by the sixth moving step portion 23f. Subsequently, as shown in Fig. 8C, the pair of movable plates 23 moves leftward. Thereafter, as shown in Fig. 8D, the pair of movable plates 23 are lowered at the left end position of the movable range. Accordingly, the test paper C_{n + 1} is placed on the seventh horizontal step portion 21g (conveyed by one pitch). The test paper Cₙ is placed on the discharge surface 20Bb. After the pair of movable plates 23 are lowered, the test paper Cₙ slides down on the discharge surface 20Bb and is put into the disposal box 60.

As described above, the discharge unit 20B discharges the test paper C that has been captured (read) by the imaging device 30 into the disposal box 60.

Next, the take-out of the disposal box 60 will be described with reference to Figs. 1, 2, and 9. For example, the fact that a predetermined amount (for example, several hundred sheets) of test papers C is stored in the disposal box 60 is detected by a sensor that detects an accumulation height of the test papers C (an upper surface position of the mountain of the test papers C). The controller 90 notifies the user of the fact through the display 5b, a speaker, or the like based on information received from the sensor (for example, when the height is equal to or greater than a predetermined threshold). For example, a message such as "The capacity of the disposal box is insufficient. Please discard the test paper." is displayed, or a beep sound notifying the same is issued. At this time, the operator stops the urine test device 1, for example, and shifts to an operation of taking out the disposal box 60.

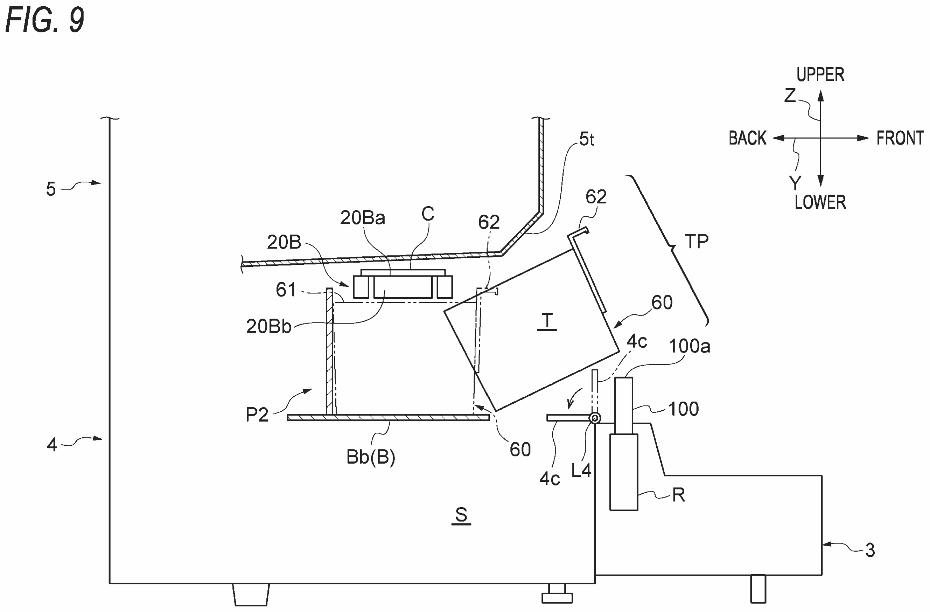

As shown in Fig. 1, the front panel 5c that is openable and closable around the axis L5 extending in the up-down direction (the Z-direction) is provided on the front surface of the upper housing 5a. A shielding cover 4c that is openable and closable around an axis L4 extending in the left-right direction (the X-direction), for example, is provided on a front surface of the lower housing 4a. First, the operator opens the front panel 5c. Subsequently, as shown in Fig. 9, the operator tilts (rotates about 90 degrees) the shielding cover 4c to fix the shielding cover 4c in a horizontal posture. The shielding cover 4c is biased in a standing direction by a spring (not shown), but the shielding cover 4c is horizontally fixed by a lock mechanism (not shown). The shielding cover 4c maintains the standing posture during the operation of the urine test device 1, thereby preventing light from entering the inside of the upper housing 5a and the lower housing 4a.

By opening the front panel 5c and making the shielding cover 4c horizontal, the take-out port T of the disposal box 60 is exposed on a front surface of the upper housings 5a and the lower housing 4a. A take-out port T is formed in front of the disposal box 60 and is larger than the disposal box 60 in a front view. The take-out port T is formed in a region surrounding the disposal box 60 in a front view. In a front view, a lower end of the take-out port T may be higher than a lower end of the disposal box 60 (the second support portion Bb of the common base B). The take-out port T extends above the upper-end edge 61 of the disposal box 60. With the above configuration, the disposal box 60 can be taken out from the take-out port T along a path TP passing above an upper end 100a (see Fig. 9) of the spit 100 on the placement table 3. The path TP is formed as a space extending forward and obliquely upward from an installation position of the disposal box 60, and there is no obstacle in the path TP. An inclined surface 5t for facilitating the take-out of the disposal box 60 may be provided inside the upper housing 5a at a position slightly in front of and above the disposal box 60. According to the inclined surface 5t, it is easy to secure the path TP that extends obliquely upward.

A handle 62 for an operator to easily grip the disposal box 60 may be provided on an upper front surface of the disposal box 60.

In the urine test device 1 of the present embodiment, the imaging position Pb is in a higher position than the deposition position Pa, and thus the test papers C are conveyed obliquely upward. In this way, not only a horizontal space but also a vertical space is effectively utilized to provide time for observing the change in coloration. This suppresses horizontal size of the urine test device 1 increasing.

In a case where the analyzer is installed alone, a mode in which a disposal box (a waste bin) is taken out from a side surface of the housing as in Patent Literature 1 may be effective. However, another analyzer may be installed beside the analyzer. When a plurality of analyzers are installed side by side, there is a restriction on taking out the disposal box from the side surface. That is, it is difficult to secure a space for taking out the analyzer, or the installation efficiency of the analyzer may be deteriorated by securing the space. In addition, since various devices are accommodated in the housing of the analyzer, an increase in size of the device is unavoidable to realize a configuration in which the disposal box is taken out from above. On the other hand, according to the urine test device 1 of the present embodiment, the disposal box 60 can be taken out from the take-out port T on the front surfaces of the lower housing 4a and the upper housing 5a. Another analyzer may be installed in the vicinity of a side. In a configuration in which the disposal box 60 is taken out from the front surface, it is easy to avoid interference with various devices in the lower housing 4a and the upper housing 5a, and an increase in size of the device can be prevented. The spit 100 may be placed on the placement table 3 in front of the device main body 2. Also in this case, since the disposal box 60 can be taken out along the path TP passing above the upper end 100a of the spit 100, interference with the spit 100 can be prevented. As a result, the disposal box 60 can be taken out from the housings 4a and 5a without any trouble while preventing an increase in size of the device.

Further, the device main body 2 includes the first conveying mechanism 10 and the second conveying mechanism 20 related to the conveyance of the test paper C, and can automatically perform the deposition and the reading. After the test paper C is read by the imaging device 30, the test paper C is put into the disposal box 60, whereby a series of operations related to the analysis (that is, handling of the test paper) is automated.

Since the discharge unit 20B is provided in the second conveying mechanism 20, processes from the reading of the test paper C to the input (discharge) into the disposal box 60 can be performed with a simple device configuration.

The test paper C is conveyed obliquely upward by the second conveying mechanism 20, and the test paper C is disposed at the imaging position Pb set at a high position. Since the disposal box 60 for receiving the test paper C can be installed at a high position, the disposal box 60 can be easily taken out at the high position. Accordingly, interference with the spit 100 placed on the placement table 3 can be easily avoided (see Fig. 9).

Further, the front panel 5c and the shielding cover 4c can be closed during the operation of the device, and the front panel 5c and the shielding cover 4c can be opened when the disposal box 60 is taken out. Since the front panel 5c and the shielding cover 4c are opened in different directions, the take-out port T and the path TP (the path TP directed forward and obliquely upward) can be easily secured. The disposal box 60 is installed beside the imaging device 30. By closing the front panel 5c and the shielding cover 4c during the operation of the device, it is possible to block the entry of light from the outside and to perform imaging (reading) by the imaging device 30 with higher accuracy.

Although the embodiment of the present disclosure has been described above, the present disclosure is not limited to the above embodiment.

The present invention may be applied to an analyzer other than the urine test device. The present invention can be applied to any device as long as the device performs analysis using a test paper and stores the analyzed test paper in a disposal box in a housing. However, the analyzer includes a placement table on which a plurality of containers are placed, and a device main body adjacent to the rear of the placement table. A take-out port of the disposal box is provided on a front surface of the housing of the device main body. The disposal box can be taken out along a path passing above an upper end of the container from the take-out port. Accordingly, even in another type of analyzer, the disposal box can be taken out without any trouble.

A device other than the imaging device using the camera 32 may be applied as the reading device. For example, the test paper C may be irradiated with light by a light irradiation unit including an LED or the like, and the reflected light may be detected by a light detection unit including a photodiode or the like. Any known device may be applied as a reading device capable of reading a color development reaction in the test paper C as optical information.

Three or more door members corresponding to the take-out port may be provided on the front surface of the housing. Only one door member corresponding to the take-out port may be provided on the front surface of the housing. The shielding cover 4c may be omitted. The door member may not be of a type capable of opening and closing (rotating), but may be detachable from the housing.

A discharge unit that discharges a test paper may be provided separately from the second conveying mechanism 20. At least one of the first conveying mechanism 10 and the second conveying mechanism 20 may be omitted.

## Claims

1. An analyzer that deposits a sample on a test paper (C) to analyze a specific component in the sample, the analyzer comprising:
a deposition device (9) configured to deposit the sample on the test paper (C);
a reading device (30) configured to read optical information of the test paper (C) on which the sample is deposited;
a conveying mechanism (20) configured to convey the test paper (C) from a deposition position (Pa) at which deposition is performed by the deposition device (9) to a reading position (Pb) at which reading by the reading device (30) is performed; and
a housing (4a, 5a) configured to accommodate the deposition device (9), the reading device (30), and the conveying mechanism (10, 20),
**characterized in that**
the reading position (Pb) is in a higher position than the deposition position (Pa), and
the conveying mechanism (20) is configured to convey the test paper obliquely upward.

2. The analyzer according to claim 1, further comprising:
a disposal box (60) into which the test paper (C) is put after the reading device (30) reads the test paper (C); and
a take-out port (T) of the disposal box (60) that is provided on a front surface of the housing (4a, 5a), wherein
the disposal box (60) is removable from the take-out port (T).

3. The analyzer according to claim 2, wherein
a plurality of door members (4c, 5c) are provided on the front surface of the housing (4a, 5a), the plurality of door members (4c, 5c) corresponding to the take-out port (T) and being openable and closable around an axis (L4, L5) extending in different directions.

4. The analyzer according to claim 3, wherein
the plurality of door members (4c, 5c) include a first door member (5c) and a second door member (4c),
the first door member (5c) is configured to open and close the housing (5a) by rotating around an axis (L5) extending in an up-down direction,
the second door member (4c) is configured to take a standing posture in the up-down direction by rotating around an axis (L4) extending in a left-right direction.

5. The analyzer according to claim 3 or 4, wherein
the deposition device (9) has a nozzle (9a) configured to collect the sample from a container (100) accommodating the sample to deposit the sample on the test paper (C), and
the nozzle (9a) is arranged in a position that does not overlap the plurality of door members (4c, 5c) in a front view.

6. The analyzer according to claim 2, further comprising a placement table (3) on which the container (100) is placed, wherein
the disposal box (60) is removable from the take-out port (T) via a path (TP) passing above the container (100) on the placement table (3).

7. The analyzer according to claim 2, further comprising a sensor configured to detect an amount of test papers (C) in the disposal box (60), wherein
the deposition device (9) is configured to perform a predetermined notification when the sensor detects that the amount of test papers (C) in the disposal box (60) is more than a predetermined amount.

## Patentansprüche

1. Analysator, der eine Probe auf ein Testpapier (C) aufbringt, um eine bestimmte Komponente in der Probe zu analysieren, wobei der Analysator umfasst:
eine Aufbringvorrichtung (9), die dazu eingerichtet ist, die Probe auf das Testpapier (C) aufzubringen;
eine Lesevorrichtung (30), die dazu eingerichtet ist, optische Informationen des Testpapiers (C), auf das die Probe aufgebracht ist, zu lesen;
einen Fördermechanismus (20), der dazu eingerichtet ist, das Testpapier (C) von einer Aufbringposition (Pa), an der das Aufbringen durch die Aufbringvorrichtung (9) erfolgt, zu einer Leseposition (Pb), an der das Lesen durch die Lesevorrichtung (30) erfolgt, zu transportieren; und
ein Gehäuse (4a, 5a), das dazu eingerichtet ist, die Aufbringvorrichtung (9), die Lesevorrichtung (30) und den Fördermechanismus (10, 20) aufzunehmen,
**dadurch gekennzeichnet, dass**
sich die Leseposition (Pb) in einer höheren Position befindet als die Aufbringposition (Pa), und
der Fördermechanismus (20) dazu eingerichtet ist, das Testpapier schräg nach oben zu transportieren.

2. Analysator nach Anspruch 1, ferner umfassend:
einen Sammelbehälter (60), in den das Testpapier (C) eingebracht wird, nachdem die Lesevorrichtung (30) das Testpapier (C) gelesen hat; und
eine an einer Vorderseite des Gehäuses (4a, 5a) vorgesehene Entnahmeöffnung (T) für den Sammelbehälter (60),
wobei
der Sammelbehälter (60) durch die Entnahmeöffnung (T) entnehmbar ist.

3. Analysator nach Anspruch 2, wobei
an der Vorderseite des Gehäuses (4a, 5a) mehrere Türelemente (4c, 5c) vorgesehen sind, wobei die mehreren Türelemente (4c, 5c) der Entnahmeöffnung (T) entsprechen und um eine Achse (L4, L5), die sich in unterschiedlichen Richtungen erstreckt, geöffnet und geschlossen werden können.

4. Analysator nach Anspruch 3, wobei
die mehreren Türelemente (4c, 5c) ein erstes Türelement (5c) und ein zweites Türelement (4c) umfassen,
das erste Türelement (5c) dazu eingerichtet ist, das Gehäuse (5a) durch Drehen um eine sich in einer Oben-Unten-Richtung erstreckende Achse (L5) zu öffnen und zu schließen, und
das zweite Türelement (4c) dazu eingerichtet ist, durch Drehen um eine sich in einer Links-Rechts-Richtung erstreckende Achse (L4) eine aufrechte Stellung in der Oben-Unten-Richtung einzunehmen.

5. Analysator nach Anspruch 3 oder 4, wobei
die Aufbringvorrichtung (9) eine Düse (9a) aufweist, die dazu eingerichtet ist, die Probe aus einem die Probe enthaltenden Behälter (100) zu entnehmen, um die Probe auf das Testpapier (C) aufzubringen, und
die Düse (9a) in einer Position angeordnet ist, in der sie sich in einer Vorderansicht nicht mit den mehreren Türelementen (4c, 5c) überlappt.

6. Analysator nach Anspruch 2, ferner umfassend einen Platzierungstisch (3), auf dem der Behälter (100) platziert ist, wobei
der Sammelbehälter (60) durch die Entnahmeöffnung (T) entlang eines Weges (TP), der oberhalb des Behälters (100) auf dem Platzierungstisch (3) verläuft, entnehmbar ist.

7. Analysator nach Anspruch 2, ferner umfassend einen Sensor, der dazu eingerichtet ist, eine Menge von Testpapieren (C) in dem Sammelbehälter (60) zu erfassen, wobei
die Aufbringvorrichtung (9) dazu eingerichtet ist, eine vorbestimmte Benachrichtigung auszugeben, wenn der Sensor erfasst, dass die Menge von Testpapieren (C) in dem Sammelbehälter (60) größer als eine vorbestimmte Menge ist.

## Revendications

1. Analyseur qui dépose un échantillon sur un papier de test (C) afin d'analyser un composant spécifique présent dans l'échantillon, l'analyseur comprenant :
un dispositif de dépôt (9) configuré pour déposer l'échantillon sur le papier de test (C) ;
un dispositif de lecture (30) configuré pour lire des informations optiques du papier de test (C) sur lequel l'échantillon est déposé ;
un mécanisme de transport (20) configuré pour transporter le papier de test (C) depuis une position de dépôt (Pa), à laquelle le dépôt est effectué par le dispositif de dépôt (9), vers une position de lecture (Pb), à laquelle la lecture par le dispositif de lecture (30) est effectuée ; et
un boîtier (4a, 5a) configuré pour loger le dispositif de dépôt (9), le dispositif de lecture (30) et le mécanisme de transport (10, 20),
**caractérisé en ce que**
la position de lecture (Pb) est située à une position plus élevée que la position de dépôt (Pa), et
le mécanisme de transport (20) est configuré pour transporter le papier de test obliquement vers le haut.

2. L'analyseur selon la revendication 1, comprenant en outre :
un bac de collecte (60) dans lequel le papier de test (C) est déposé après que le dispositif de lecture (30) a lu le papier de test (C) ; et
une ouverture de retrait (T) du bac de collecte (60) qui est prévue sur une face avant du boîtier (4a, 5a), dans lequel
le bac de collecte (60) peut être retiré par l'ouverture de retrait (T).

3. L'analyseur selon la revendication 2, dans lequel
une pluralité d'éléments de porte (4c, 5c) est prévue sur la face avant du boîtier (4a, 5a), ladite pluralité d'éléments de porte (4c, 5c) correspondant à l'ouverture de retrait (T) et pouvant être ouverts et fermés autour d'un axe (L4, L5) s'étendant dans des directions différentes.

4. L'analyseur selon la revendication 3, dans lequel
la pluralité d'éléments de porte (4c, 5c) comprend un premier élément de porte (5c) et un second élément de porte (4c),
le premier élément de porte (5c) est configuré pour ouvrir et fermer le boîtier (5a) en tournant autour d'un axe (L5) s'étendant dans une direction haut-bas,
le second élément de porte (4c) est configuré pour adopter une position dressée dans la direction haut-bas en tournant autour d'un axe (L4) s'étendant dans une direction gauche-droite.

5. L'analyseur selon la revendication 3 ou 4, dans lequel
le dispositif de dépôt (9) comporte une buse (9a) configurée pour prélever l'échantillon dans un récipient (100) contenant l'échantillon afin de déposer l'échantillon sur le papier de test (C), et
la buse (9a) est disposée dans une position dans laquelle elle ne se superpose pas à la pluralité d'éléments de porte (4c, 5c) en vue de face.

6. L'analyseur selon la revendication 2, comprenant en outre une table de pose (3) sur laquelle est placé le récipient (100), dans lequel
le bac de collecte (60) peut être retiré par l'ouverture de retrait (T) le long d'un trajet (TP) passant au-dessus du récipient (100) sur la table de pose (3).

7. L'analyseur selon la revendication 2, comprenant en outre un capteur configuré pour détecter une quantité de papiers de test (C) dans le bac de collecte (60), dans lequel
le dispositif de dépôt (9) est configuré pour émettre une notification prédéterminée lorsque le capteur détecte que la quantité de papiers de test (C) dans le bac de collecte (60) est supérieure à une quantité prédéterminée.
